# EUROPEAN PATENT APPLICATION

(11) **EP 4 548 890 A1**
(43) Date of publication of application: **07.05.2025**
(21) Application number: 23928765.9
(22) Date of filing: 16.11.2023
(51) Int. Cl.: A61F 13/532

(54) **UNDERPANTS-STYLE DISPOSABLE WEARABLE ARTICLE**

(30) Priority: 17.03.2023 JP 2023043217
(71) Applicant: Daio Paper Corporation, Ehime 799-0492 (JP)
(72) Inventor: NAKAMARU, Hikari, Shikokuchuo-shi, Ehime 799-0113 (JP); FUJIWARA, Yuto, Shikokuchuo-shi, Ehime 799-0113 (JP)
(74) Representative: Williams Powell
(86) International application number: PCT/JP2023/041205
(87) International publication number: WO 2024/195196

(57) **Abstract**

To suppress deformation at an end portion of an absorber on a waist opening side due to an elastic member.

The above problem is solved by a configuration in which an absorber 56 has an edge region 56E including the whole of an edge on a waist opening WO side and a portion adjacent thereto on a crotch portion M side in a width direction WD, the entire edge region 56E is located in a fixing region 201 for an inner member 200 and is fixed to outer members 12F and 12B, a groove 53 recessed from a surface of the absorber 56 into the absorber 56 and having a bottom portion 51 compressed in a thickness direction is formed in a pattern that is continuous in the width direction WD over the entire edge region 56E in the width direction WD, and in the edge region 56E, the thickness of the bottom portion 51 of the groove 53 is constant and an area ratio of the bottom portion 51 of the groove 53 increases as it goes from the center of the absorber 56 in the width direction WD toward both side edges of the absorber 56.

## Description

### Technical Field

The present invention relates to an underpants-type disposable wearable article excellent in shape maintainability of an inner member.

### Background Art

In general, an underpants-type disposable wearable article such as an underpants-type disposable diaper or an underpants-type sanitary product includes: an outer member having a front lower torso portion, a back lower torso portion, and side seals formed by bonding both side portions of the front lower torso portion and the back lower torso portion; and an inner member attached to the outer member so as to extend from the front lower torso portion to the back lower torso portion, and both side portions of the front lower torso portion and both side portions of the back lower torso portion are bonded to each other to form the side seals, whereby a waist opening and a pair of left and right leg openings are formed (see, for example, Patent Literature 1).

An elastic member is disposed in the lower torso portion of the outer member, whereby a stretchable region that contracts in the width direction in a natural length state and is extensible in the width direction is formed. With this stretchable region, the lower torso portion fits well to a body surface of a wearer.

The inner member includes an absorber that continues from the front lower torso portion to the back lower torso portion through a crotch portion, and a liquid content of an excrement is absorbed and held by the absorber.

In general, the front lower torso portion and the back lower torso portion each have a fixing region for fixing the inner member to the outer member, and substantially the entire region of the absorber overlapping the outer member is fixed to the outer member. Therefore, when the stretchable regions of the front lower torso portion and the back lower torso portion overlap the absorber, not only elasticity of the stretchable regions is limited by rigidity of the absorber, but also the absorber receives a contraction force in the width direction, whereby there is a possibility that twisting or cracking is likely to occur, absorption performance is deteriorated, or wearing feeling or appearance is deteriorated.

For this reason, conventionally, in general, for example, an elongated elastic member is continuously attached to both a first region including a portion overlapping the absorber and a portion between the overlapped portion and the side seals in the front lower torso portion and the back lower torso portion and a second region between the first region and the waist opening in the width direction over the entire outer member in the front-back direction, and substantially the entire elastic member in the portion overlapping the absorber is finely cut in the first region, whereby a non-stretchable region in which a contraction force in the width direction does not act (a contraction force is killed) is formed in the portion overlapping the absorber, and the other regions are stretchable regions. As a result, in the first region, a region having the absorber is the non-stretchable region, and a side seal side of the non-stretchable region (portion close to the side seal) is an intermittent stretchable band of the stretchable region, and in the second region, the entire region from one of the side seals to the other of the side seals is a continuous stretchable band of the stretchable region.

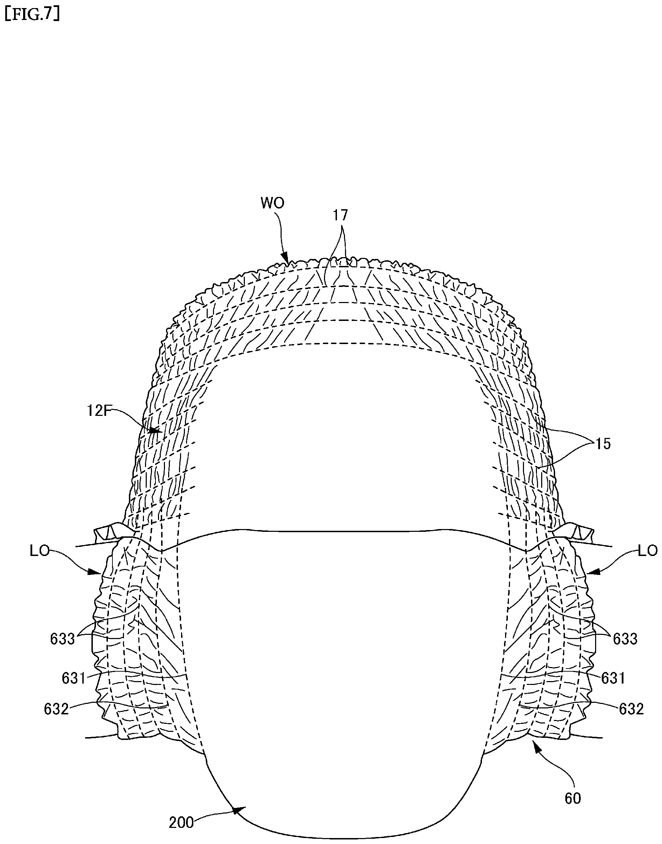

However, in such an underpants-type disposable wearable article, as illustrated in Fig. 7 in a natural length state, both corner portions of the absorber on the waist opening side selectively contract in the width direction, an intermediate portion in the width direction at an end portion of the absorber on the waist opening side hardly contracts, and the intermediate portion in the width direction at the end portion of the absorber on the waist opening side has a shape bulging toward a back surface side (external surface side). The underpants-type disposable wearable article has a problem that compactness (decrease in thickness) at the time of product packaging or product carrying is hindered, or the underpants-type disposable wearable article appears to a user as a low-quality product.

### Citation List

### Patent Literature

Patent Literature 1: JP 2019-150655 A

### Summary of Invention

### Technical Problem

Therefore, a main object of the present invention is, for example, to provide an underpants-type disposable wearable article that suppresses deformation at an end portion of an absorber on a waist opening side due to an elastic member.

### Solution to Problem

An underpants-type disposable wearable article that has solved the above problem is as follows.

### <First Aspect>

An underpants-type disposable wearable article including:
an outer member having a front lower torso portion, a back lower torso portion, and side seals formed by bonding both side portions of the front lower torso portion and both side portions of the back lower torso portion to each other;
an inner member extending from the front lower torso portion to the back lower torso portion through a crotch portion;
a waist opening formed by a front edge of the front lower torso portion and a back edge of the back lower torso portion; and
leg openings formed on both sides of the inner member, wherein
the outer member has a fixing region where the inner member is fixed to at least the front lower torso portion and the back lower torso portion,
the inner member includes an absorber that continues from a position overlapping the front lower torso portion to a position overlapping the back lower torso portion,
the absorber has an edge region including a whole of an edge on a waist opening side and a portion adjacent thereto on a crotch portion side in a width direction, and the entire edge region is located in the fixing region of the inner member and fixed to the outer member,
in the front lower torso portion and the back lower torso portion, a first region including a portion overlapping the absorber and a portion between the overlapped portion and the side seals is an intermittent stretchable band having a non-stretchable region provided at least at an intermediate portion in the width direction in the portion overlapping the absorber and a stretchable region provided between the non-stretchable region and each of the side seals, and a second region located between the intermittent stretchable band and the waist opening is a continuous stretchable band in which a stretchable region is continuous in the width direction from one of the side seals to the other of the side seals,
the stretchable region in the intermittent stretchable band and the continuous stretchable band incorporates an elastic member and is stretchable between a natural length state in which the stretchable region contracts in the width direction together with the elastic member and a stretched state in which the stretchable region extends in the width direction together with the elastic member,
a groove recessed from a surface of the absorber into the absorber and having a bottom portion compressed in a thickness direction is formed in a pattern continuous in the width direction over the entire edge region in the width direction, and
in the edge region, a thickness of the bottom portion of the groove is constant and an area ratio of the bottom portion of the groove increases, the area ratio of the bottom portion of the groove is constant and the thickness of the bottom portion of the groove decreases, or the area ratio of the bottom portion of the groove increases and the thickness of the bottom portion of the groove decreases as it goes from a center of the absorber in the width direction toward both side edges of the absorber.

### (Action and effect)

When a groove recessed from a surface of the absorber into the absorber and having a bottom portion compressed in the thickness direction is formed in a predetermined pattern, since the compressed bottom portion has high density and high rigidity, shape maintainability of the edge region is improved, and deformation at an end portion of the absorber on a waist opening side due to an elastic member can be suppressed.

However, if the shape maintainability is improved at a uniform area ratio of the bottom portion of the groove and a uniform thickness of the bottom portion over the entire edge region, flexibility of the edge region of the absorber may decrease.

On the other hand, when rigidity of the edge region is increased stepwise or continuously as it goes from the center of the absorber in the width direction toward both side edges of the absorber by changing at least one of the area ratio of the bottom portion of the groove and the thickness of the bottom portion as in the present aspect, the shape maintainability of the edge region of the absorber increases as it approaches both corner portions. Therefore, it is possible to suppress deformation at an end portion of the absorber on a waist opening side due to an elastic member while suppressing a decrease in flexibility of the edge region of the absorber, which is preferable.

### <Second Aspect>

The underpants-type disposable wearable article according to the first aspect, wherein
the edge region is a region where the groove is continuous in a lattice shape, and
each of a thickness of the bottom portion of the groove and a width of the bottom portion of the groove in the edge region is constant, and an area of a unit frame including the groove in the edge region gradually decreases as it goes from a center in the width direction toward both side edges.

### (Action and effect)

When a groove recessed from a surface of the absorber into the absorber and having a bottom portion compressed in the thickness direction is formed in the absorber in a lattice-shaped continuous pattern, the bottom of the groove having high density and high rigidity continues in a lattice shape, whereby the shape maintainability of the region having the groove is particularly high. Here, although the shape maintainability changes depending on the thickness of the bottom portion of the groove of the absorber, a change width thereof is not large, an influence on absorption performance such as liquid diffusibility is large, and ease of manufacturing is also deteriorated. In addition, when the width of the bottom portion of the groove is increased, feel of the hard bottom portion of the groove is easily transmitted to the skin. Therefore, it is preferable to gradually decrease the area of the unit frame including the groove as it goes from the center in the width direction toward both side edges while making each of the thickness of the bottom portion of the groove and the width of the bottom portion of the groove constant as in the present aspect. As a result, the shape maintainability of the edge region of the absorber increases as it approaches both corner portions, and therefore it is possible to suppress deformation at an end portion of the absorber on a waist opening side due to an elastic member while suppressing a decrease in flexibility of the edge region of the absorber.

### <Third Aspect>

The underpants-type disposable wearable article according to the first or second aspect, wherein
a plurality of the elastic members in the continuous stretchable band are attached at intervals in a front-back direction and are elongated elastic members in the width direction, and
an interval between the elongated elastic member closest to the edge region of the absorber and the edge region in a front-back direction is 15 mm or less.

### (Action and effect)

In the underpants-type disposable wearable article, an elongated elastic member is often used as a means for adding elasticity to a lower torso portion of the outer member. In this case, according to findings of the present inventor, when the interval in the front-back direction between the elastic member closest to the edge region of the absorber and the edge region is 15 mm or less, the end portion of the absorber on the waist opening side is particularly easily deformed. Therefore, in such a case, when the rigidity of the edge region is increased stepwise or continuously as it goes from the center of the absorber in the width direction toward both side edges of the absorber, it is particularly significant.

### <Fourth Aspect>

The underpants-type disposable wearable article according to the third aspect, wherein
when a test piece obtained by cutting a portion of the continuous stretchable band up to 15 mm from the edge region of the absorber to the waist opening side from one of the side seals to the other of the side seals is extended to 90% of a maximum width in an unfolded state and then contracted to 50% of the maximum width in the unfolded state, a contraction force is 0.4 N or more.

### (Action and effect)

According to the findings of the present inventor, in the case of the present aspect, the end portion of the absorber on the waist opening side is particularly easily deformed. Therefore, in such a case, when the rigidity of the edge region is increased stepwise or continuously as it goes from the center of the absorber in the width direction toward both side edges of the absorber, it is particularly significant.

### <Fifth Aspect>

The underpants-type disposable wearable article according to any one of the first to fourth aspects, wherein
the absorber includes one or more layers formed by mixing and accumulating pulp fibers and super absorbent polymer particles,
a basis weight of the pulp fibers in the absorber is 80 to 450 g/m²,
a weight ratio of pulp fibers : super absorbent polymer particles in the absorber is 20 : 80 to 80 : 20,
a maximum value of a thickness of the absorber is 1 to 20 mm,
a thickness of the bottom portion is 5 to 40% of a maximum value of the thickness of the absorber, and
a width of the bottom portion is 1 to 3 mm.

### (Action and effect)

The size of the groove can be appropriately determined, but in the absorber as in the present aspect, it is preferable to form the groove within the range of the present aspect.

### <Sixth Aspect>

The underpants-type disposable wearable article according to any one of the first to fifth aspects, wherein
the groove is formed in an oblique lattice shape including a first portion inclined clockwise by 40 to 60° in plan view with respect to a front-back direction and a second portion inclined counterclockwise by 40 to 60° in plan view with respect to the front-back direction.

### (Action and effect)

A pattern of the groove can be appropriately determined, but is preferably an oblique lattice shape as in the present aspect.

### Advantageous Effects of Invention

As described above, according to the present invention, for example, there is an advantage that deformation at an end portion of an absorber on a waist opening side due to an elastic member can be suppressed.

### Brief Description of Drawings

Fig. 1 is a plan view illustrating a front surface of an underpants-type disposable diaper in a state where the diaper is unfolded.
Fig. 2 is a plan view illustrating a back surface of the underpants-type disposable diaper in a state where the diaper is unfolded.
Fig. 3 is a cross-sectional view taken along line 3-3 in Fig. 1.
Fig. 4 is a cross-sectional view taken along line 4-4 in Fig. 1.
Fig. 5 is a cross-sectional view taken along line 5-5 in Fig. 1.
Fig. 6 is a plan view illustrating a surface of an inner member.
Fig. 7 is a perspective view of the underpants-type disposable diaper as viewed from obliquely lower front.
Fig. 8 is a cross-sectional view of a main part illustrating a worn state.
Fig. 9 is a cross-sectional view of an absorbent element.
Fig. 10 is a plan view of the absorbent element.
Fig. 11 is an enlarged plan view illustrating a main part of Fig. 10.
Fig. 12 is a plan view of an absorbent element.
Fig. 13 is a plan view of the absorbent element.
Fig. 14(a) is a photograph of a sample A of the underpants-type disposable diaper, and Fig. 14(b) is a photograph of a sample B of the underpants-type disposable diaper.

### Description of Embodiments

Hereinafter, the above-described underpants-type disposable wearable article will be described in detail with reference to an example of an underpants-type disposable diaper illustrated in the attached drawings. Note that components adjacent in a thickness direction are fixed or bonded to each other as necessary in a similar manner to a known diaper also at a portion other than a fixed or bonded portion described below. A dotted pattern portion in a cross-sectional view indicates an adhesive such as a hot melt adhesive as a fixing or bonding means. The hot melt adhesive can be applied by a known method such as slot application, bead application into a continuous line or a dot shape, spray application into a spiral shape, a Z shape, or a wave shape (regular or irregular), or pattern coating (transfer of the hot melt adhesive by a letterpress method). Alternatively or in addition, in a fixed portion of an elastic member, the hot melt adhesive can be applied to an outer peripheral surface of an elastic member, and the elastic member can be fixed to an adjacent member. Examples of the hot melt adhesive include an EVA-based agent, a pressure sensitive adhesive rubber-based agent (elastomer-based agent), a polyolefin-based agent, and a polyester/polyamide-based agent, and these can be used without particular limitation. As a fixing or bonding means for bonding components to each other, a means by material welding such as heat sealing or ultrasonic sealing can also be used. In a portion where a liquid pervious property in a thickness direction is required, components adjacent to each other in a thickness direction are fixed or bonded to each other in an intermittent pattern. For example, when such intermittent fixing or bonding is performed with a hot melt adhesive, intermittent pattern application in a spiral shape, a Z shape, a wave shape, or the like can be suitably used, and when application is performed in a range equal to or larger than an application width by one nozzle, intermittent pattern application in a spiral shape, a Z shape, a wave shape, or the like can be performed with or without an interval in a width direction. As the bonding means for bonding constituent members to each other, a means by material welding such as heat sealing or ultrasonic sealing can also be used.

As a nonwoven fabric in the following description, a known nonwoven fabric can be appropriately used according to a site or a purpose. Examples of a constituent fiber of the nonwoven fabric include, but are not limited to, a synthetic fiber such as a polyolefin-based fiber including polyethylene and polypropylene, a polyester-based fiber, or a polyamide-based fiber (including a composite fiber such as core-sheath in addition to a single component fiber), a regenerated fiber such as rayon or cupra, and a natural fiber such as cotton. These fibers can be mixed and used. In order to enhance flexibility of the nonwoven fabric, it is preferable to use a crimped fiber as the constituent fiber. In addition, the constituent fiber of the nonwoven fabric may be a hydrophilic fiber (including a fiber that has become hydrophilic by a hydrophilizing agent), a hydrophobic fiber, or a water-repellent fiber (including a fiber that has become water-repellent by a water repellent agent). In addition, the nonwoven fabric is generally classified into a short fiber nonwoven fabric, a long fiber nonwoven fabric, a spunbonded nonwoven fabric, a meltblown nonwoven fabric, a spunlace nonwoven fabric, a thermal bond (air through) nonwoven fabric, a needle punch nonwoven fabric, a point bond nonwoven fabric, a laminated nonwoven fabric (including an SMS nonwoven fabric, an SMMS nonwoven fabric, or the like having a meltblown layer sandwiched between spunbond layers), and the like depending on a fiber length, a sheet forming method, a fiber bonding method, and a stacked structure, and any of these nonwoven fabrics can be used.

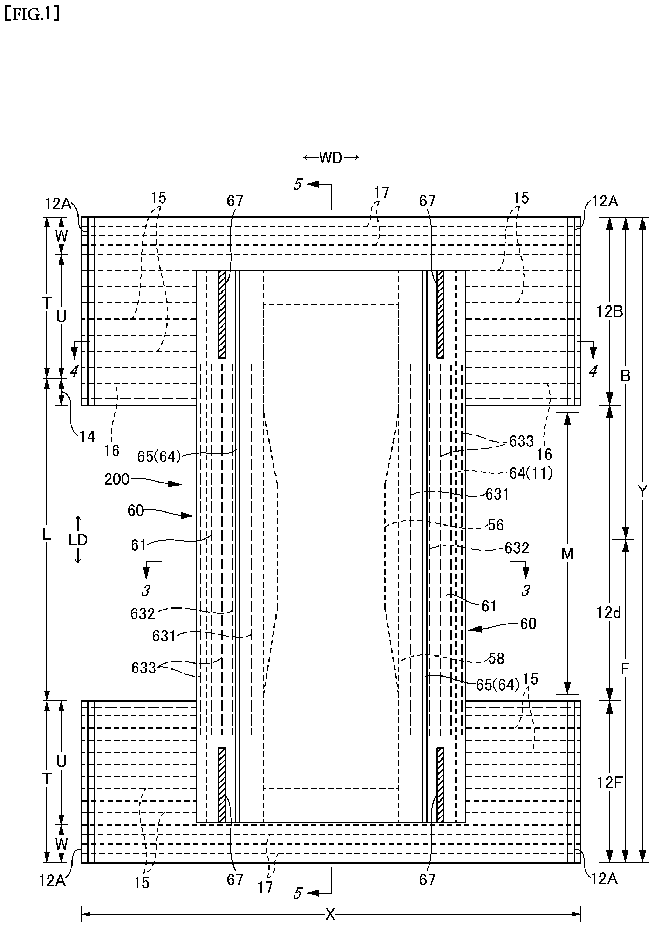

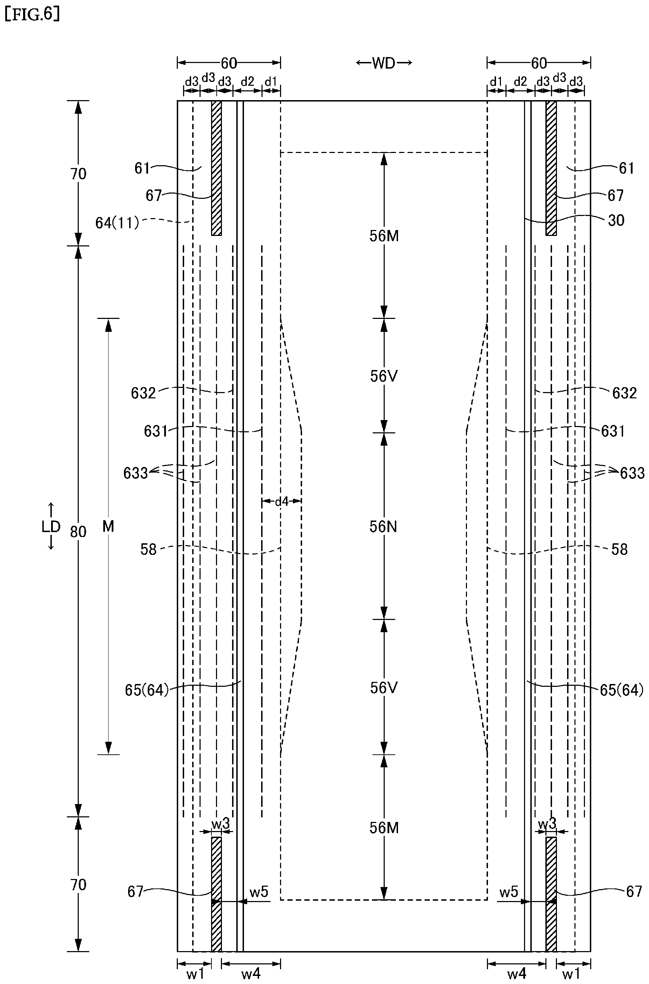

Figs. 1 to 7 illustrate an example of an underpants-type disposable diaper. A present underpants-type disposable diaper 100 includes: a rectangular front outer member 12F constituting a front lower torso portion FT; a rectangular back outer member 12B constituting a back lower torso portion BT; and an inner member 200 bonded to a middle of each of the outer members 12F and 12B in a width direction WD so as to extend from the front outer member 12F to the back outer member 12B through a crotch portion M. Both side portions of the front outer member 12F and both side portions of the back outer member 12B are bonded to each other to form side seals 12A. As a result, an opening formed by a front edge and a back edge of the outer members 12F and 12B is a waist opening WO through which a wearer's torso passes, and a portion surrounded by lower edges of the outer members 12F and 12B and a side edge of the inner member 200 on each side of the inner member 200 in the width direction WD is a leg opening LO through which the leg passes. The inner member 200 is a portion for absorbing and holding excrement such as urine, and the outer members 12F and 12B are portions for supporting the inner member 200 with respect to the body of a wearer. A reference character Y indicates the maximum length of the diaper in an unfolded state (front-back direction length from an edge of the waist opening WO of the front body F to an edge of the waist opening WO of the back body B), and a reference character X indicates the maximum width of the diaper in an unfolded state.

The present underpants-type disposable diaper 100 has a lower torso region T defined as a front-back direction range (front-back direction range from the waist opening WO to an upper end of the leg opening LO) having the side seals 12A, and an intermediate region L defined as a front-back direction range of a portion forming the leg opening LO (between a front-back direction region having the side seals 12A of the front body F and a front-back direction region having the side seals 12A of the back body B). A portion located in the lower torso region T in each of the front outer member 12F and the back outer member 12B, that is, each of the front lower torso portion FT and the back lower torso portion BT can be conceptually separated into a "waist portion" W that forms an edge portion of a waist opening and an "under-waist portion" U that is a portion lower than the waist portion W. Usually, in a case where each of the front lower torso portion FT and the back lower torso portion BT has a boundary in which a stretching stress in the width direction WD changes (for example, a fineness or a stretch rate of an elastic member changes), a portion closer to the waist opening WO than the boundary closest to the waist opening WO is the waist portion W. In a case where there is no such a boundary, a portion extending so as to be closer to the waist opening WO than an absorber 56 or the inner member 200 is the waist portion W. The front-back direction length varies depending on the size of a product and can be appropriately determined. For example, the length of the waist portion W can be 15 to 40 mm, and the length of the under-waist portion U can be 65 to 120 mm. Meanwhile, both side edges of the intermediate region L are each narrowed in a substantially U shape or a curved shape so as to follow a periphery of a wearer's leg, and the wearer's legs pass therethrough. As a result, the underpants-type disposable diaper in an unfolded state has a substantially hourglass shape as a whole.

### (Fixing region of inner member)

The inner member 200 can be fixed to the outer members 12F and 12B by a bonding means by material welding such as heat sealing or ultrasonic sealing, or with a hot melt adhesive HM3. In the illustrated example, a back surface of the inner member 200 is fixed to inner surfaces of the outer members 12F and 12B via the hot melt adhesive HM3. In the illustrated example, the fixing region 201 of the inner member 200 is provided in a region where the front lower torso portion FT and the back lower torso portion BT overlap the inner member 200. The fixing region of the inner member is preferably provided over substantially the entire region except for a peripheral edge of the region where the outer members 12F and 12B overlap the inner member 200.

### (Outer member)

As in the illustrated example, the outer members 12F and 12B include the rectangular front outer member 12F which is a portion constituting at least the front lower torso portion FT (lower torso portion of the front body F) and the rectangular back outer member 12B which is a portion constituting at least the back lower torso portion BT (lower torso portion of the back body B). The front outer member 12F and the back outer member 12B may be separated from each other in the front-back direction LD without being continuous on a crotch side (outer member separated type), or may be continuous from the front body F to the back body B through the crotch portion M (outer integral type) although not illustrated. A separation distance 12d in the front-back direction in the outer member separated type can be, for example, about 40 to 60% of the maximum length Y. In the illustrated example, lower edges of the front outer member 12F and the back outer member 12B are linear in the width direction WD, but a lower edge of at least one of the front outer member 12F and the back outer member 12B may be curved along a periphery of the leg.

In the outer member separated type underpants-type disposable diaper, the inner member 200 is exposed between the front outer member 12F and the back outer member 12B. Therefore, in order to prevent a liquid impervious sheet 11 from being exposed to a back surface of the inner member 200, a cover nonwoven fabric layer 13 extending from a portion between the front outer member 12F and the inner member 200 to a portion between the back outer member 12B and the inner member 200 is preferably provided on the back surface of the inner member 200. An inner surface and an outer surface of the cover nonwoven fabric layer 13 can be bonded to facing surfaces thereof via a second hot melt adhesive HM2 and a third hot melt adhesive HM3, respectively. As a nonwoven fabric used for the cover nonwoven fabric layer 13, for example, a similar material to that of the outer members 12F and 12B can be appropriately selected.

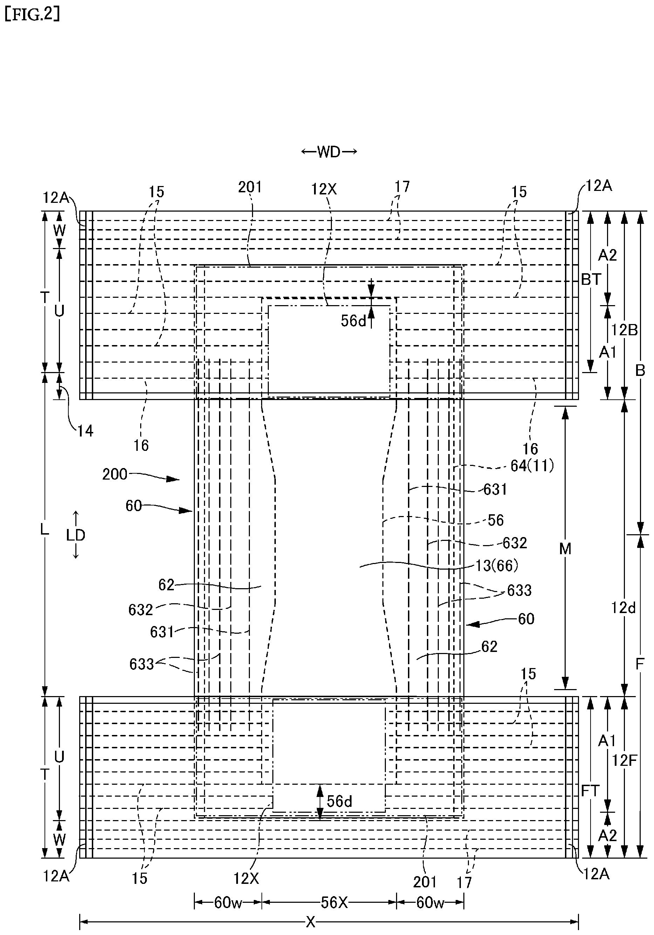

In the example illustrated in Figs. 1 and 2, the size of the back outer member 12B in the front-back direction is longer than that of the front outer member 12F, the front outer member 12F does not have a portion corresponding to the intermediate region L, and the back outer member 12B has a gluteal cover portion 14 extending from a lower torso region T toward the intermediate region L. However, the size of the front outer member 12F in the front-back direction LD may be equal to that of the back outer member 12B, and each of the front outer member 12F and the back outer member 12B does not have to have a portion corresponding to the intermediate region L.

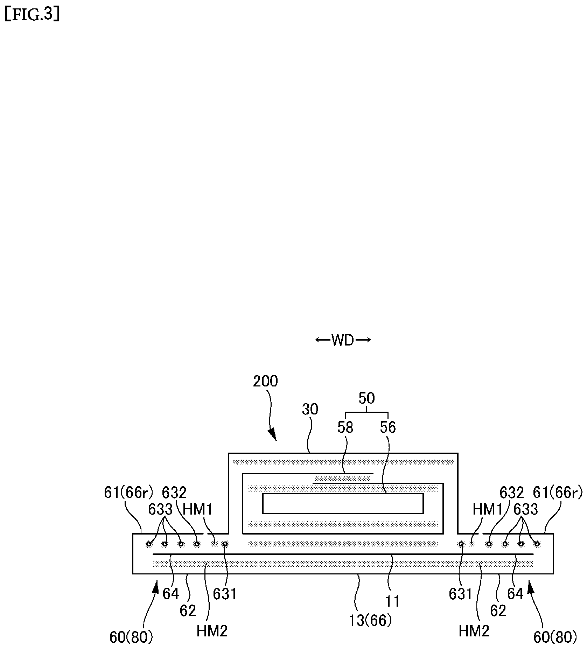

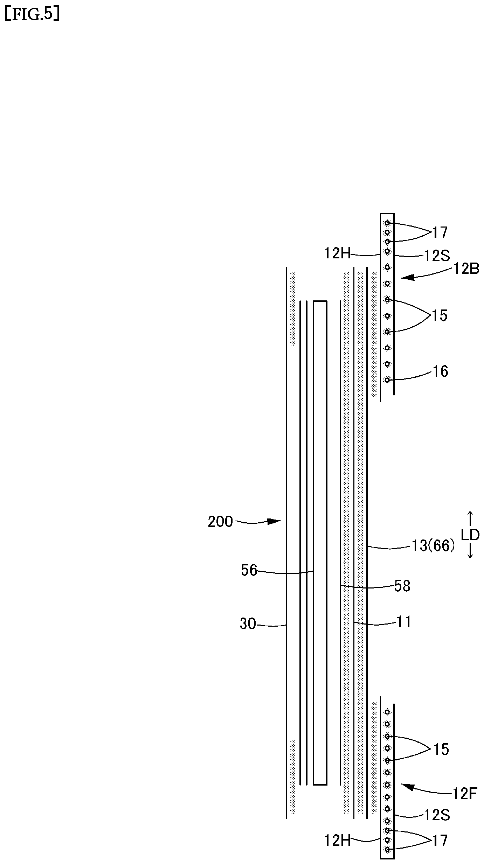

As illustrated in Figs. 3 to 5, the outer members 12F and 12B are formed by bonding an outer sheet layer 12S and an inner sheet layer 12H adjacent to an outer side and an inner side of elastic members 15 to 17 described later, respectively, by a bonding means such as a hot melt adhesive or welding. As illustrated in Fig. 5, the outer sheet layer 12S and the inner sheet layer 12H can be formed by folding a single sheet of a sheet material such that a fold is located on the waist opening side. In addition, the outer sheet layer 12S and the inner sheet layer 12H can be formed by sticking two sheets of a sheet material to each other although not illustrated.

As the sheet material used for the outer sheet layer 12S and the inner sheet layer 12H, any material can be used without particular limitation, but a nonwoven fabric is preferable. In a case where a nonwoven fabric is used, the nonwoven fabric preferably has a basis weight of about 8 to 20 g/m² per sheet.

In the outer members 12F and 12B, in order to enhance fitting of a wearer with respect to a lower torso, the elastic members 15 to 17 are attached between the outer sheet layer 12S and the inner sheet layer 12H at a predetermined stretch rate, and a stretchable region that elastically stretches and contracts in the width direction WD together with the elastic members 15 to 17 is formed. The stretchable region contracts together with the elastic member 15 to 17, and can stretch and contract between a natural length state in which the stretchable region contracts together with the elastic member 15 to 17 to form wrinkles or pleats and a stretched state in which the stretchable region is stretched in the width direction to a predetermined stretch rate at which the stretchable region is stretched without wrinkles together with the elastic members 15 to 17. As the elastic members 15 to 17, in addition to an elongated elastic member (illustrated example) such as a rubber thread, a known elastic member such as a belt-shaped member, a net-shaped member, or a film-shaped member can be used without particular limitation. As the elastic members 15 to 17, either a synthetic rubber or a natural rubber may be used. For sticking the outer sheet layer 12S and the inner sheet layer 12H in the outer members 12F and 12B and fixing the elongated elastic members 15 to 17 sandwiched therebetween, at least one of a hot melt adhesive by various application methods and a fixing means by material welding such as heat sealing or ultrasonic sealing can be used. When the entire surfaces of the outer members 12F and 12B are fixed rigidly, softness is impaired. Therefore, preferably, a portion other than a bonded portion of the elongated elastic members 15 to 17 is not bonded or weakly bonded. In the illustrated example, by applying a hot melt adhesive only to outer peripheral surfaces of the elongated elastic members 15 to 17 by an application means such as a comb gun or a SureWrap nozzle, and sandwiching the elongated elastic members 15 to 17 between both the sheet layers 12S and 12H, the elongated elastic members 15 to 17 are fixed to both the sheet layers 12S and 12H only with the hot melt adhesive applied to the outer peripheral surfaces of the elongated elastic members 15 to 17, and both the sheet layers 12S and 12H are fixed to each other.

The illustrated elastic members 15 to 17 will be described in more detail. Between the outer sheet layer 12S and the inner sheet layer 12H in the waist portion W of each of the outer members 12F and 12B, a plurality of waist elastic members 17 are attached at intervals in the front-back direction and in an extended state in the width direction at a predetermined stretch rate, and a waist stretchable region continuous over the entire width direction WD is formed. One or more waist elastic members 17 disposed in a region adjacent to the under-waist portion U may overlap the inner member 200, or may be disposed on each side thereof in the width direction WD except for a central portion in the width direction overlapping the inner member 200. As the waist elastic member 17, it is preferable to dispose 2 to 15 rubber threads, particularly 4 to 10 rubber threads each having a fineness of 300 to 940 dtex, particularly about 470 to 780 dtex (in a case of a synthetic rubber. A cross section area of 0.05 to 1.5 mm², particularly about 0.1 to 1.0 mm² in a case of a natural rubber) at intervals of 2 to 12 mm, particularly 3 to 7 mm. A stretch rate of the waist portion W in the width direction WD due to the waist elastic member 17 is preferably 150 to 300%, and particularly preferably about 220 to 280%. In the waist portion W, all of the elastic members in the front-back direction LD do not have to have the same fineness and the same stretch rate. For example, the fineness and the stretch rate of the elastic member may be partially different.

Between the outer sheet layer 12S and the inner sheet layer 12H in the under-waist portion U of each of the outer members 12F and 12B, a plurality of under-waist elastic members 15 formed of an elongated elastic member are attached at intervals in the front-back direction and in an extended state in the width direction at a predetermined stretch rate, and an under-waist stretchable region is formed on each side in the width direction WD excluding a central portion in the width direction overlapping the inner member 200. As the under-waist elastic member 15, it is preferable to dispose 5 to 30 rubber threads each having a fineness of 300 to 940 dtex, particularly about 470 to 780 dtex (in a case of a synthetic rubber. A cross section area of 0.05 to 1.5 mm², particularly about 0.1 to 1.0 mm² in a case of a natural rubber) at intervals of 1 to 15 mm, particularly 3 to 8 mm. A stretch rate of the under-waist portion U in the width direction WD due to the under-waist elastic member 15 is preferably 200 to 350%, and particularly preferably about 240 to 300%. In the under-waist portion U, all of the elastic members in the front-back direction LD do not have to have the same fineness and the same stretch rate. The fineness and the stretch rate of the elastic member may be partially different.

Between the outer sheet layer 12S and the inner sheet layer 12H in the gluteal cover portion 14 of the back outer member 12B, one or more gluteal cover elastic members 16 formed of an elongated elastic member are attached at intervals in the up-down direction and in an extended state in the width direction at a predetermined stretch rate on each side in the width direction WD excluding a central portion in the width direction overlapping the inner member 200, and a gluteal cover stretchable region is formed on each side in the width direction WD excluding a central portion in the width direction overlapping the inner member 200. As the gluteal cover elastic member 16, it is preferable to fix 2 to 10 rubber threads each having a fineness of 300 to 940 dtex, particularly about 470 to 780 dtex (in a case of a synthetic rubber. A cross section area of 0.05 to 1.5 mm², particularly about 0.1 to 1.0 mm² in a case of a natural rubber) at intervals of 5 to 40 mm, particularly 5 to 20 mm at a stretch rate of 150 to 300%, particularly 180 to 260%.

In the front lower torso portion FT and the back lower torso portion BT, the first region including a portion overlapping the absorber 56 and a portion between the overlapped portion and the side seals 12A is preferably an intermittent stretchable band A1 having a non-stretchable region 12X provided at least at an intermediate portion in the width direction WD in the portion overlapping the absorber 56 and a stretchable region provided between the non-stretchable region 12X and each of the side seals 12A, and the second region located between the intermittent stretchable band A1 and the waist opening WO is preferably a continuous stretchable band A2 in which a stretchable region is continuous in the width direction WD from one of the side seals 12A to the other of the side seals 12A.

The intermittent stretchable band A1 in the illustrated example may extend beyond the range of the first region (beyond the absorber 56) to the waist opening WO side as long as the intermittent stretchable band A1 includes the first region. In the illustrated example, in the back lower torso portion BT, only the first region is the intermittent stretchable band A1, and a portion between the first region and the waist opening WO is the continuous stretchable band A2, and in the front lower torso portion FT, a region including a portion overlapping the fixing region 201 of the inner member 200 and a portion between the overlapped portion and the side seal 12A (a region extending to the waist opening WO side beyond the first region) is the intermittent stretchable band A1. However, the present invention is not limited thereto. Therefore, these may be reversed. Alternatively, a region including a portion overlapping the fixing region 201 of the inner member 200 and a portion between the overlapped portion and the side seal 12A may be the intermittent stretchable band A1 in each of the front lower torso portion FT and the back lower torso portion BT, or only the first region may be the intermittent stretchable band A1 in each of the front lower torso portion FT and the back lower torso portion BT. In addition, the intermittent stretchable band A1 of the illustrated example is formed in the range including only the under-waist stretchable region having the under-waist elastic member 15 in the front-back direction LD, but the range may be expanded to the waist opening WO side so as to include a part of the waist stretchable region having the waist elastic member 17.

The continuous stretchable band A2 in the illustrated example is formed only by the waist stretchable region including the waist elastic member 17 in the front lower torso portion FT, and formed by the entire waist stretchable region including the waist elastic member 17 and a part of the under-waist stretchable region including the under-waist elastic member 15 in the back lower torso portion BT. However, the present invention is not limited thereto. Therefore, these may be reversed. Alternatively, the continuous stretchable band A2 in each of the front lower torso portion FT and the back lower torso portion BT may be formed only by the waist stretchable region having the waist elastic members 17, or may be formed by the entire waist stretchable region having the waist elastic members 17 and a part of the under-waist stretchable region having the under-waist elastic members 15.

As illustrated in the drawing, when the first region including the portion overlapping the absorber 56 and the portion between the overlapped portion and the side seal 12A is not the continuous stretchable band A2 but the intermittent stretchable band A1, the absorber 56 does not contract more than necessary in the width direction WD, fitting does not deteriorate, a poor appearance such as a lumpy appearance is not obtained, and absorbability does not deteriorate. Such an intermittent stretchable band A1 includes not only a case where the elastic members 15 and 16 are present only on both sides in the width direction WD, but also a case where the elastic members 15 and 16 are present from one side to the other side in the width direction WD across the absorber 56, but the intermediate portion or the entire portion in the width direction overlapping the absorber 56 is the non-stretchable region 12X with almost no contraction force due to fine cutting of the elastic members 15 and 16 or the like (which is substantially equivalent to not providing the elastic members 15 and 16), and both sides thereof in the width direction WD are stretchable regions (contraction force acting portions). The non-stretchable region 12X is preferably located between both side edges of the inner member 200, and more preferably located between both side edges of the absorber 56. The size of the non-stretchable region 12X in the width direction WD can be appropriately determined, but is preferably 0.6 to 1.2 times, and more preferably 0.8 to 1.0 times the maximum width of the absorber 56. In addition, the size of the non-stretchable region 12X in the width direction WD is preferably 0.5 to 1.0 times, and more preferably 0.6 times to 0.8 times the size of the continuous stretchable band A2 in the width direction WD.

### (Inner member)

The shape of the inner member 200 is arbitrary, but is a rectangle in the illustrated example. As illustrated in Figs. 3 to 5, the inner member 200 includes a liquid pervious top sheet 30 to be located on a wearer's skin side, a liquid impervious sheet 11, and the absorbent element 50 interposed therebetween. A reference character 60 indicates a side flap 60 having a planar gather.

### (Top sheet)

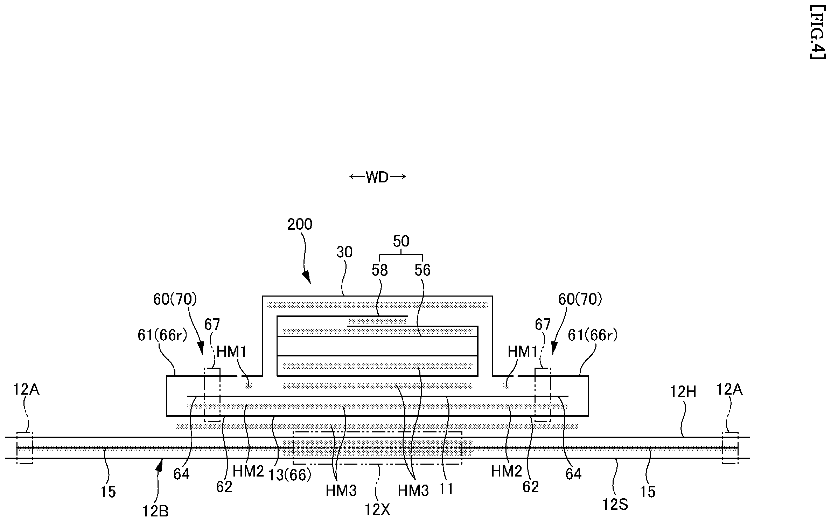

As the top sheet 30, a liquid pervious material such as a perforated or imperforated nonwoven fabric or a perforated plastic sheet can be used without particular limitation. However, in a case where the top sheet 30 also serves as a cover material of a liquid impervious sheet layer 64 as illustrated in Figs. 3 and 4, the top sheet 30 is suitably made of a nonwoven fabric.

Both sides of the top sheet 30 may be folded back to a back surface side of the absorbent element 50 at a side edge of the absorbent element 50 or may protrude from the side edge of the absorbent element 50 to a lateral side without being folded back. In addition, if necessary, the maximum width of the top sheet 30 may be made shorter than the maximum width of the absorbent element 50, and both side edges of the top sheet 30 may be located on the absorbent element 50.

### (Intermediate sheet)

Although not illustrated, in order to rapidly transfer a liquid that has passed through the top sheet 30 to the absorber, it is possible to dispose an intermediate sheet (also referred to as "second sheet") having a higher liquid permeation speed than the top sheet 30. The intermediate sheet is used in order to rapidly transfer a liquid to the absorber to enhance absorption performance of the absorber, and to prevent a "returning" phenomenon of the absorbed liquid from the absorber. The intermediate sheet can be omitted.

### (Liquid impervious sheet)

A material of the liquid impervious sheet 11 disposed on a back surface side of the absorber 56 is not particularly limited, but examples thereof include a plastic film formed of a polyolefin-based resin such as polyethylene or polypropylene. For the liquid impervious sheet 11, it is preferable to use a liquid impervious and moisture pervious material that has been favorably used from a viewpoint of preventing stuffiness in recent years. As a moisture pervious plastic film, a microporous plastic film obtained by kneading an inorganic filler in a polyolefin-based resin such as polyethylene or polypropylene, molding a sheet, and then stretching the sheet in a monoaxial or biaxial direction is widely used.

As in the example illustrated in Figs. 3 and 4, the liquid impervious sheet 11 can be configured to extend to the inside of the side flap to improve a waterproof property of a planar gather, can have a width (not illustrated) to fit on a back surface side of the absorbent element 50, or can be folded back to a front surface side at a side edge of the absorbent element 50 to extend to a portion between the absorbent element 50 and the top sheet 30.

### (Absorbent element)

As illustrated in Figs. 3, 4, and 9, the absorbent element 50 includes the absorber 56 and a wrapping sheet 58 wrapping the entire absorber 56. The wrapping sheet 58 can be omitted.

### (Absorber)

The absorber 56 can be formed by an assembly of fibers. As this fiber assembly, in addition to those obtained by accumulating a short fiber such as fluff pulp or a synthetic fiber, a filament assembly obtained by opening a tow (fiber bundle) of a synthetic fiber such as cellulose acetate as necessary can also be used. In a case where fluff pulp or a short fiber is accumulated, a fiber basis weight can be, for example, about 80 to 450 g/m², and more preferably about 80 to 200 g/m². In a case of a filament assembly, a fiber basis weight can be, for example, about 30 to 120 g/m². In a case of a synthetic fiber, a fineness is, for example, 1 to 16 dtex, preferably 1 to 10 dtex, and more preferably 1 to 5 dtex.

Super absorbent polymer particles are preferably mixed in the fiber assembly. The super absorbent polymer particles include "powder" in addition to "particles". As the super absorbent polymer particles, those used for this type of disposable diaper can be used as they are. For example, when sieving using a standard sieve of 500 µm (JIS Z8801-1: 2006) (shake for five minutes) is performed, particles in which a ratio of particles remaining on the sieve is 30% by weight or less are desirable. When sieving using a standard sieve of 180 µm (JIS Z8801-1: 2006) (shake for five minutes) is performed, particles in which a ratio of particles remaining on the sieve is 60% by weight or more are desirable.

A material of the super absorbent polymer particles can be used without particular limitation, but those having a water absorption capacity (JIS K7223-1996 "Test method for water absorption capacity of super absorbent resin") of 40 g/g or more are suitable. Examples of the super absorbent polymer particles include a starch-based material, a cellulose-based material, and a synthetic polymer-based material. A starch-acrylic acid (salt) graft copolymer, a saponified product of a starch-acrylonitrile copolymer, a cross-linked product of sodium carboxymethyl cellulose, an acrylic acid (salt) polymer, or the like can be used. As the shapes of the super absorbent polymer particles, a usually used particulate material shape is suitable, but other shapes can also be used.

As the super absorbent polymer particles, those having a water absorption speed of 70 seconds or less, particularly 40 seconds or less are suitably used. When the absorption speed is too slow, so-called returning that liquid supplied into the absorber 56 returns out of the absorber 56 tends to occur.

As the super absorbent polymer particles, those having a gel strength of 1000 Pa or more are suitably used. This makes it possible to effectively suppress a sticky feeling after liquid absorption even in a case of using the bulky absorber 56.

The basis weight of the super absorbent polymer particles can be appropriately determined depending on the absorption amount required for an application of the absorber 56. Therefore, the basis weight can be 50 to 350 g/m² although this cannot be applied generally. The basis weight of a polymer of less than 50 g/m² makes it difficult to ensure the absorption amount. The basis weight of more than 350 g/m² saturates an effect.

A ratio between the fibers and the super absorbent polymer particles in the absorber 56 is not particularly limited, and for example, a weight ratio of pulp fibers : super absorbent polymer particles can be 20 : 80 to 80 : 20, and more preferably 40 : 60 to 60 : 40. In particular, when the weight ratio of pulp fibers : super absorbent polymer particles is 40 : 60 to 60 : 40, a thinner absorber 56 can be obtained when comparison is performed at the same area and the same absorption amount. A thickness 56t of the absorber 56 is not particularly limited, but can be 1 to 20 mm, and more preferably 2 to 5 mm.

The absorber 56 continues from the front lower torso portion FT to the back lower torso portion BT. In the absorber 56, the entire edge region 56E (preferably the entire absorber 56) including the whole of at least an edge on the waist opening WO side and a portion adjacent thereto on the crotch portion M side in the width direction WD is preferably located in the fixing region 201 of the inner member 200 and fixed to the outer members 12F and 12B via the wrapping sheet 58, the liquid impervious sheet 11, the hot melt adhesive HM3, and the like. Note that a reference character 56X indicates a maximum width of the absorber 56.

In order to easily ensure the absorption amount in the crotch portion M, the absorber 56 preferably has a substantially rectangular shape. However, as illustrated in Figs. 1, 2, and 10, when the crotch portion M has a minimum width portion 56N and has a widened portion 56V in which the width gradually increases from the minimum width portion 56N to a maximum width portion 56M on a front side of the crotch portion M and from the crotch portion M to the maximum width portion 56M on a back side of the crotch portion M, fitting of the inner thigh is improved by the narrowing portions 56N and 56V including the minimum width portion 56N and the widened portion 56V, which is preferable. In the latter case, in order to ensure the absorption amount at the crotch portion M, the size of the minimum width portion 56N in the width direction WD is preferably 0.8 times or more the maximum width 56X of the absorber 56. The size of each of the narrowing portions 56N and 56V in the front-back direction is preferably about 20 to 30% of the maximum length Y of the product.

Note that, in a case where the absorber 56 has the narrowing portions 56N and 56V described above, the crotch portion M means a range having the narrowing portions 56N and 56V in the front-back direction LD, in a case where the absorber 56 does not have the narrowing portions 56N and 56V but an outer shape in an unfolded state has a narrowing portion as in the illustrated example, the crotch portion M means a range having the narrowing portion of the outer shape in the front-back direction LD (in the case of the illustrated example, a portion between the front outer member 12F and the back outer body 12B), and in a case where the absorber 56 has no narrowing portion, the crotch portion M means a portion located at the center in the front-back direction LD and having a size in the front-back direction LD of 20 to 30% of the maximum length Y of the product.

### (Wrapping sheet)

In a case where the wrapping sheet 58 is used, as a material thereof, a liquid pervious material such as tissue paper, particularly, crepe paper, a nonwoven fabric, a polylaminated nonwoven fabric, or a sheet with small holes can be used. Note that it is desirable that the wrapping sheet 58 is a sheet from which super absorbent polymer particles do not escape. In a case where a nonwoven fabric is used instead of crepe paper, a hydrophilic nonwoven fabric having a dense layer is suitable. As such a nonwoven fabric, a laminated nonwoven fabric (SMS nonwoven fabric, SSMMS nonwoven fabric, or the like) in which one or more meltblown layers and one or more protective layers for protecting the meltblown layers, such as a spunbond layer, are laminated is particularly suitable, and polypropylene, a polyethylene/polypropylene composite material, or the like can be used as a material thereof. A nonwoven fabric having a basis weight of 5 to 40 g/m², particularly of 8 to 20 g/m² is desirable.

A wrapping structure of the wrapping sheet 58 can be determined appropriately. However, a structure is preferable in which the wrapping sheet 58 is wound around the absorber 56 cylindrically so as to surround front and back surfaces and both side surfaces of the absorber 56, the front and back end portions of the wrapping sheet 58 are caused to project from the front and back of the absorber 56, and the projecting portions are crushed in a thickness direction and bonded by a bonding means such as a hot melt adhesive from viewpoints of ease of manufacture, prevention of leakage of the super absorbent polymer particles from front and back edges, and the like.

### (Side flap)

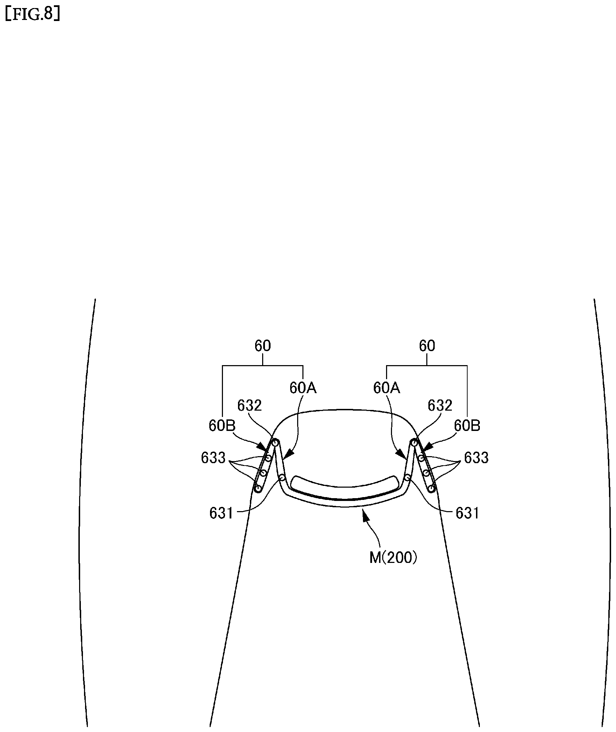

As illustrated in Figs. 1 to 4, 7, and 8, the inner member 200 preferably has a side flap 60. The side flaps 60 are a pair of portions extending from a side edge of the absorber 56 to both sides in the width direction WD, and each extend in the front-back direction LD from a region overlapping a lower torso stretchable region of the front body F (a region including the under-waist elastic member 15 in the illustrated example) to a region overlapping a lower torso stretchable region of the back body B (a region including the under-waist elastic member 15 in the illustrated example). The side flap 60 includes a side nonwoven fabric 66 extending from a front surface of the side flap 60 to a back surface of the side flap 60 through a side edge of the side flap 60, and elongated gather elastic members 631 to 633 disposed in the front-back direction LD, for example, between a front surface portion 61 and a back surface portion 62 of the side flap 60. The front surface portion 61 and the back surface portion 62 extend over the entire side flap 60 in the front-back direction LD. In the illustrated example, the front surface portion 61 is formed by a folded portion 66r of the side nonwoven fabric 66. However, the present invention is not limited thereto. A front end portion and a back end portion of the side flap 60 do not contract in the front-back direction LD together with the gather elastic members 631 to 633, and form a side non-stretchable region 70 having a fixed portion 67 in which the front surface portion 61 is fixed to the back surface portion 62. A portion between the front and back side non-stretchable regions 70 in the side flap 60 is a planar gather region 80 to which the gather elastic members 631 to 633 are fixed, which contracts in the front-back direction LD together with the gather elastic members 631 to 633, and which is extensible in the front-back direction LD together with the gather elastic members 631 to 633. The present underpants-type disposable diaper does not include three-dimensional gathers rising on a front surface side, and includes only planar gathers of the side flap 60. In the fixed portion 67 in the illustrated example, the front surface portion 61 is indirectly fixed to the back surface portion 62 via the liquid impervious sheet layer 64, but is directly fixed in a case where another member such as the liquid impervious sheet layer 64 is not interposed.

The size of the side non-stretchable region 70 in the front-back direction LD at a front end portion of the side flap 60 may be different from or the same as that of a back end portion as in the illustrated example. Note that a reference character 60w in the drawing indicates the size of the side flap 60 in the width direction WD. In addition, a reference character w1 indicates a distance (first distance) in the width direction WD between the fixed portion 67 and a side edge of the side non-stretchable region 70, a reference character w2 indicates a size (second distance) in the width direction WD of a portion not fixed to the outer member 12 in each side portion (side flap) of the inner member, a reference character w3 indicates a size of the fixed portion 67 in the width direction WD, a reference character w4 indicates a distance (third distance) in the width direction WD between the fixed portion 67 and a side edge of the absorber 56, and a reference character w5 indicates a distance (fourth distance) in the width direction WD between the fixed portion 67 and a central edge of the front surface portion 61 in the width direction WD.

As the gather elastic members 631 to 633, an elongated elastic member such as a thread-shaped rubber or a belt-shaped rubber can be used. When a rubber thread is used, the gather elastic members 631 to 633 have a fineness of preferably 400 to 950 dtex, more preferably 470 to 780 dtex. The gather elastic members 631 to 633 have a stretch rate of preferably 200 to 320%, more preferably 240 to 320%. In the drawing, a reference character 631 indicates a first gather elastic member located first from the absorber 56 side, a reference character 632 indicates a second gather elastic member located second, and a reference character 633 indicates a third gather elastic member located third or later. In addition, a reference character d1 indicates an interval (first interval) in the width direction WD between a side edge of the portion 56M having the maximum width of the absorber 56 and the first gather elastic member 631, a reference character d2 indicates an interval (second interval) in the width direction WD between the first gather elastic member 631 and the second gather elastic member 632, and a reference character d3 indicates an interval in the width direction WD between the second gather elastic member 632 and the third gather elastic member 633 and an interval (third interval) in the width direction WD between the adjacent third gather elastic members 633.

In the side flap 60, for sticking layers adjacent to each other in the thickness direction or fixing the gather elastic members 631 to 633 sandwiched therebetween, at least one of a hot melt adhesive by various application methods and a fixing means by material welding such as heat sealing or ultrasonic sealing can be used. When the entire surfaces of layers adjacent to a front surface side and a back surface side of the gather elastic members 631 to 633 are stuck, softness is impaired. Therefore, preferably, a portion other than a bonded portion of the gather elastic members 631 to 633 is not bonded or weakly bonded. In the illustrated example, by applying a hot melt adhesive only to an outer peripheral surface of the gather elastic members 631 to 633 by an application means such as a comb gun or a SureWrap nozzle, and sandwiching the gather elastic members 631 to 633 between layers adjacent to a front surface side and a back surface side of the gather elastic members 631 to 633, the gather elastic members 631 to 633 are fixed to the layers adjacent to a front surface side and a back surface side of the gather elastic members 631 to 633, and the layers adjacent to a front surface side and a back surface side of the gather elastic members 631 to 633 are fixed to each other only with the hot melt adhesive applied to the outer peripheral surface of the gather elastic members 631 to 633. In a case where there is an end portion of a nonwoven fabric or a sheet away from the gather elastic members 631 to 633 like an end portion of the top sheet 30 in the illustrated example, a hot melt adhesive (the first hot melt adhesive HM1 in the illustrated example) can be separately applied to the end portion of the nonwoven fabric or the sheet in order to fix them.

As the side nonwoven fabric 66, a product obtained by subjecting a soft nonwoven fabric having excellent uniformity and concealability, such as a spunbonded nonwoven fabric (SS, SSS, or the like), an SMS nonwoven fabric (SMS, SSMMS, or the like), or a melt blown nonwoven fabric, to a water repellent treatment with silicon or the like as necessary can be used suitably. The side nonwoven fabric 66 preferably has a fiber basis weight of about 10 to 30 g/m². In the example illustrated in Figs. 3 and 4, as can be seen from the fact that a front surface nonwoven fabric layer closer to a base edge than the nonwoven fabric absent portion 65 is formed by the top sheet 30, it is also possible to make a material of each of the front surface nonwoven fabric layer and the back surface nonwoven fabric layer partially different, or it is also possible to make the materials of the front surface nonwoven fabric layer and the back surface nonwoven fabric layer different from each other.

### (Groove)

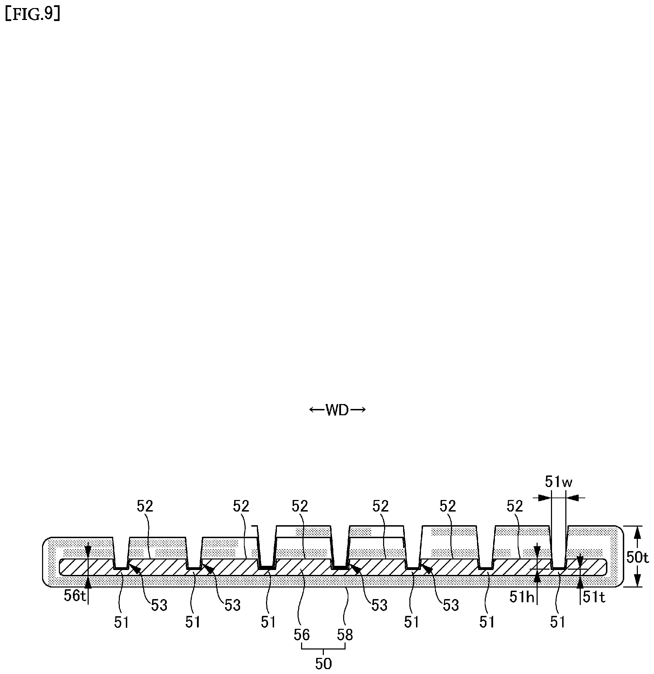

As illustrated in Figs. 9 to 11, the absorber 56 preferably has a groove 53 recessed from a surface of the absorber 56 into the absorber 56 and having a bottom portion 51 compressed in the thickness direction. The bottom portion 51 of the groove 53 is a high-density portion compressed by pressing (direct pressing) to have substantially the same thickness. Hereinafter, a portion other than the bottom portion 51 is referred to as a non-compressed portion 52. Although the non-compressed portion 52 is a portion having a larger thickness and a lower density than the bottom portion 51, even in the non-compressed portion 52, near the periphery of the bottom portion 51, the absorber 56 deforms so as to be pulled by compressive formation of the bottom portion 51. As a result, the density increases as it approaches the bottom portion 51. As long as the non-compressed portion 52 has a larger thickness and a lower density than the bottom portion 51, the entire non-compressed portion 52 may be compressed in the thickness direction at the same time as, or before or after compression processing for forming the bottom portion 51. The shape and arrangement of the non-compressed portion 52 are determined according to the shape and arrangement of the bottom portion 51.

The groove 53 of the absorber 56 may be formed by embossing only the absorber 56 without heating or with heating, or may be formed including the absorber 56 and other laminated members by performing embossing in a state where members other than the absorber 56 are laminated, such as the absorbent element 50 (that is, together with the wrapping sheet 58) and the inner member 200.

When the above-described groove 53 is formed in a pattern continuous in the width direction WD over the entire edge region 56E in the width direction WD including the whole of an edge of the absorber 56 on the waist opening WO side and a portion adjacent thereto on the crotch portion M side in the width direction WD, since the compressed bottom portion 51 has high density and high rigidity, shape maintainability of the edge region 56E is improved, and deformation at an end portion of the absorber 56 on the waist opening WO side due to an elastic member can be suppressed. However, if the shape maintainability is improved at a uniform area ratio of the bottom portion 51 of the groove 53 and a uniform thickness of the bottom portion 51 over the entire edge region 56E, flexibility of the edge region 56E of the absorber 56 may decrease. Therefore, in the edge region 56E, preferably, the thickness of the bottom portion 51 of the groove 53 is constant and the area ratio of the bottom portion 51 of the groove 53 increases as it goes from the center of the absorber 56 in the width direction WD toward both side edges of the absorber 56. Alternatively, although not illustrated, the area ratio of the bottom portion 51 of the groove 53 may be constant and the thickness of the bottom portion 51 of the groove 53 may be decreased, or the area ratio of the bottom portion 51 of the groove 53 may be increased and the thickness of the bottom portion 51 of the groove 53 may be decreased. When rigidity of the edge region 56E is increased stepwise or continuously as it goes from the center of the absorber 56 in the width direction WD toward both side edges of the absorber 56 by changing at least one of the area ratio of the bottom portion 51 of the groove 53 and the thickness of the bottom portion 51 as described above, the shape maintainability of the edge region 56E of the absorber 56 increases as it approaches both corner portions. Therefore, it is possible to suppress deformation of an end portion of the absorber 56 on the waist opening WO side due to the waist elastic member 17 and the under-waist elastic member 15 while suppressing a decrease in flexibility of the edge region 56E of the absorber 56.

The above-described groove 53 may be formed in the entire absorber 56 as long as it is formed in a pattern continuous in the width direction WD over the entire edge region 56E of the absorber 56, or may be formed only in the edge region 56E or only in a partial range in the front-back direction including the edge region 56E. In particular, as in the illustrated example, if the pattern of the groove 53 in the edge region 56E uniformly continues over the entire absorber 56 in the front-back direction LD, positioning of the grooves 53 in the front-back direction LD is unnecessary, and manufacturing is easy, which is preferable. However, the edge region 56E can have the groove 53 having a pattern different from that of a region other than the edge region 56E. In addition, the groove 53 can be formed such that a pattern in the edge region 56E of the front body F is different from that in the edge region 56E of the back body B. Furthermore, in both the edge region 56E of the front body F and the edge region 56E of the back body B, at least one of the thickness of the bottom portion 51 of the groove 53 and the area ratio of the bottom portion 51 of the groove 53 may change as described above as it goes from the center of the absorber 56 in the width direction WD toward both side edges of the absorber 56. However, in only one of the edge region 56E of the front body F and the edge region 56E of the back body B, at least one of the thickness of the bottom portion 51 of the groove 53 and the area ratio of the bottom portion 51 of the groove 53 may be changed as described above, and in the other region, the groove 53 may be formed so as to have a uniform pattern in the width direction WD, or the groove 53 does not have to be formed at all.

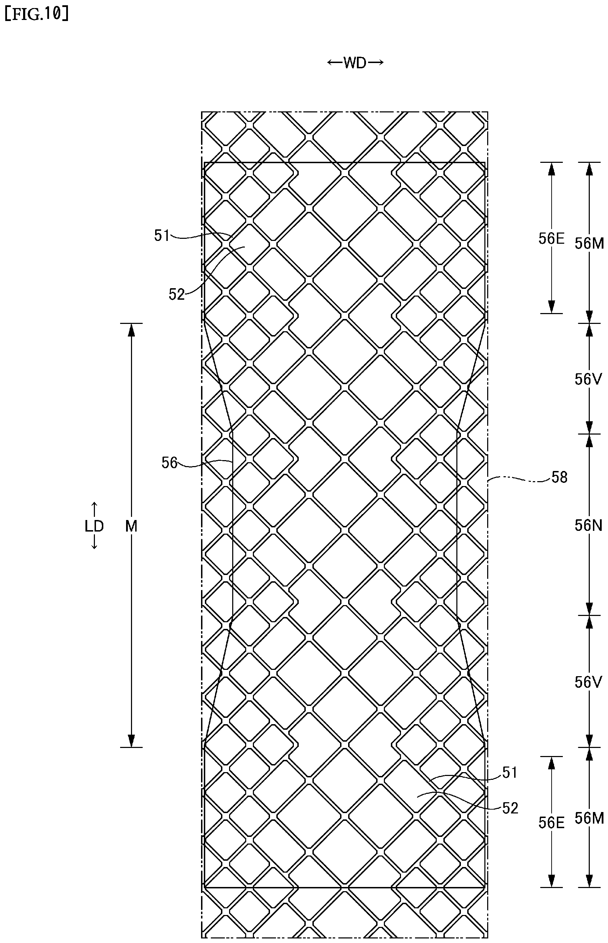

The size of the edge region 56E in the front-back direction LD can be appropriately determined as long as the size is equal to or less than the sizes of the front lower torso portion FT and the back lower torso portion BT in the front-back direction LD, the groove 53 continuous in the width direction WD is formed in the edge region 56E, and the area ratio and the like of the bottom portion 51 can be changed as it goes from the center of the absorber 56 in the width direction WD toward both side edges of the absorber 56. As an example, the size of the edge region 56E in the front-back direction LD is preferably 40 mm or more, and more preferably 60 mm or more. In addition, in a case where the grooves 53 are formed in a lattice shape as described later, when the size of the edge region 56E in the front-back direction LD is twice or more the size of the smallest unit frame in the front-back direction LD, one smallest unit frame can be necessarily present in the edge region 56E, which is preferable. When the size of the edge region 56E in the front-back direction LD is twice or more the size of the largest unit frame in the front-back direction LD, the unit frame can be necessarily present over the entire edge region 56E in the width direction WD, which is more preferable. As in the illustrated example, in a case where the pattern of the groove 53 in the edge region 56E uniformly continues over the entire absorber 56 in the front-back direction LD, the size of the edge region 56E in the front-back direction LD is equal to the sizes of the front lower torso portion FT and the back lower torso portion BT in the front-back direction LD as illustrated in Fig. 10.

The sizes, the number, and the like of the bottom portions 51 of the grooves 53 can be appropriately determined, but a depth 51h of the bottom portion 51 (thickness 56t of the absorber 56 - thickness 51t of the bottom portion of the groove) is preferably 0.5 to 5 mm, and an area ratio of the bottom portion 51 of the groove 53 (a ratio of the area of the bottom portion 51 to a surface area of the absorbent element 50) is preferably 20 to 40%.

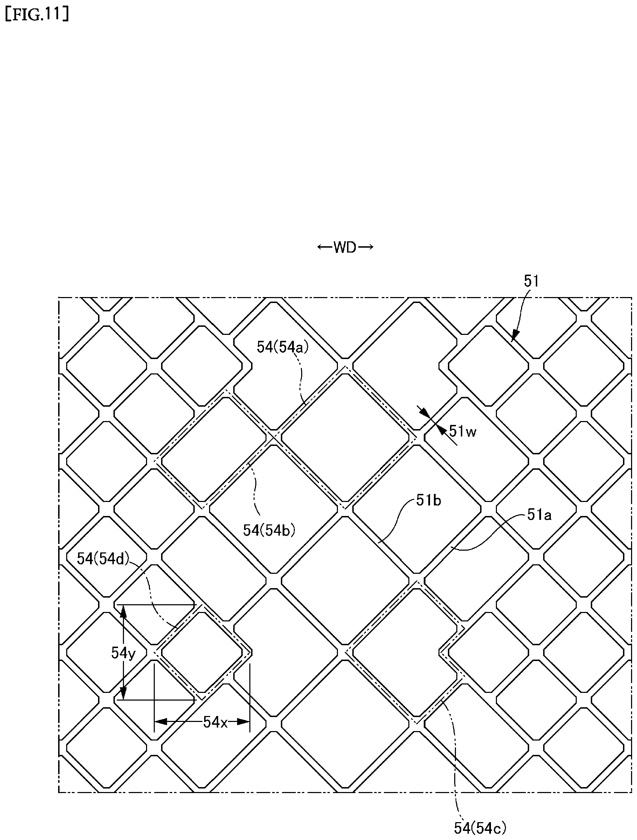

As long as the groove 53 having the bottom portion 51 is continuous in the width direction WD, it is possible to form the groove 53 in a lattice-shaped continuous pattern as illustrated in Figs. 10 and 11, or it is possible to form, along the width direction WD, only one groove 53 or a plurality of grooves 53 at intervals in the front-back direction LD, to form, along an oblique direction, only one groove 53 or a plurality of grooves 53 at intervals in a direction orthogonal to the continuous direction of the groove 53, to form one or more grooves 53 extending in a wavy line shape, to form the groove 53 in another geometric pattern, or to form the groove 53 in a continuous pattern having a motif of a character, an animal, a plant, or the like, although not illustrated. In particular, when the groove 53 is formed in a lattice-shaped continuous pattern, the pulp fibers and the super absorbent polymer particles constituting the absorber 56 are constrained in a unit frame 54 (minimum frame) including the bottom portion 51 compressed in the thickness direction, and therefore it is possible to prevent twisting and cracking of the absorber 56.

The width 51w of the bottom portion 51 of the groove 53 (the width of the bottom portion of the compressed portion formed in the absorbent element 50) can be appropriately determined, but is preferably about 1 to 3 mm in a normal case.

The thickness 51t of the bottom portion 51 of the groove 53 can be appropriately determined, but is preferably 5 to 40%, and particularly preferably 5 to 30% of a maximum value of the thickness 50t of the absorbent element 50 in a normal case.

In a case where the grooves 53 are formed in a lattice shape, the shape of the unit frame 54 is not particularly limited, and may be a substantially square shape as in the illustrated example, may be a substantially rhombic shape (excluding a square shape), and may be another polygonal shape such as a substantially rectangular shape, or a substantially triangular shape. In addition, as long as the bottom portions 51 of the grooves are formed in a lattice shape, unit frames 54 having different shapes may be included.

As in the examples illustrated in Figs. 10 and 11, one preferable lattice-shaped pattern of the groove 53 is an oblique lattice-shaped pattern including a first portion 51a extending in a direction inclined clockwise by 40 to 60° (more preferably 45 to 50°) in plan view with respect to the front-back direction LD and a second portion 51b extending in a direction inclined counterclockwise by 40 to 60° (more preferably 45 to 50°) in plan view with respect to the front-back direction LD. In this case, the unit frame 54 has a substantially rhombic shape. The angle of each of the first portion 51a and the second portion 51b with respect to the front-back direction LD is particularly preferably 45 degrees.

The size of the unit frame 54 can be appropriately determined, but if the size is too large, a rigidity improving effect is poor, and if the size is too small, the product is hard. Therefore, in a normal case, a size 54x of the unit frame 54 in the width direction WD is preferably about 15 to 20 mm. A size 54y of the unit frame 54 in the front-back direction LD is preferably about 15 to 20 mm.

When the area ratio of the bottom portion 51 of the groove 53 is changed in the width direction WD of the absorber 56, the width of the bottom portion 51 of the groove 53 can be changed while the pattern of the groove 53 is substantially constant, the pattern of the groove 53 can be changed while the width 51w of the bottom portion 51 of the groove 53 is substantially constant, or both the pattern of the groove 53 and the width 51w of the bottom portion 51 of the groove 53 can be changed. Note that when the width 51w of the bottom portion 51 of the groove 53 is increased in order to increase the area ratio of the bottom portion 51 of the groove 53, feel of the hard bottom portion 51 of the groove 53 is easily transmitted to the skin. Therefore, as in the illustrated example, it is preferable to change the pattern of the groove 53 (specifically, the number of grooves 53 per unit area) while the width 51w of the bottom portion 51 of the groove 53 is substantially constant. In addition, in a case where the thickness of the bottom portion 51 of the groove 53 is changed in the width direction WD of the absorber 56, a change width of the shape maintainability of the absorber 56 due to the change is not large, an influence on absorption performance such as liquid diffusibility is large, and ease of manufacturing is also deteriorated. Therefore, the thickness of the bottom portion 51 of the groove 53 can be changed while the area ratio of the bottom portion 51 of the groove 53 is substantially constant, but it is more preferable to change the area ratio of the bottom portion 51 of the groove 53 while the thickness of the bottom portion 51 of the groove 53 is substantially constant.

Figs. 10 to 13 illustrate some preferred examples of a pattern of the groove. In these examples, while the width 51w and the thickness of the bottom portion 51 of the groove 53 are each substantially constant, the pattern of the groove 53 changes in the width direction WD such that the area ratio of the bottom portion 51 of the groove 53 gradually increases as it goes from the center of the absorber 56 in the width direction WD toward both side edges of the absorber 56. The size of the area of each unit frame 54 can be appropriately determined, but the area of a larger unit frame out of a pair of unit frames adjacent in the width direction so as to have a common side is preferably 1.1 to 2.5 times the area of a smaller unit frame.

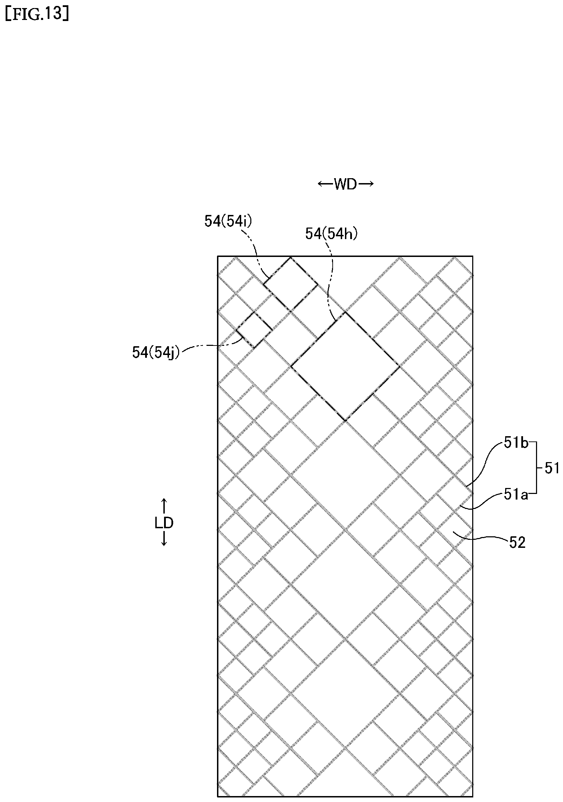

In a case where the pattern of the groove 53 has a lattice shape, as illustrated in Figs. 10, 11, and 12, by combining a plurality of types of shapes of the unit frame 54 to form a pattern in which the area of the unit frame 54 gradually decreases as it goes from the center in the width direction WD toward both side edges, it is possible to gradually increase the area ratio of the bottom portion 51 of the groove 53 as it goes from the center of the absorber 56 in the width direction WD toward both side edges of the absorber 56. In addition, as illustrated in Fig. 13, by combining the unit frames 54 having the same shape (in the illustrated example, a rhombus (square)) but different sizes to form a pattern in which the area of the unit frame 54 gradually decreases as it goes from the center in the width direction WD toward both side edges, the area ratio of the bottom portion 51 of the groove 53 can be gradually increased as it goes from the center of the absorber 56 in the width direction WD toward both side edges of the absorber 56.

More specifically, the pattern of the groove 53 in the edge region 56E of the example illustrated in Figs. 10 and 11 has an oblique lattice shape. In this pattern, rhombic first unit frames 54a having the largest area are arranged without a gap at the center in the width direction WD, rectangular second unit frames 54b in which one side is common to the first unit frame 54a and the length of a short side intersecting the common side is 2/3 of the length of the common side, and L-shaped third unit frames 54c in which two sides are common to the first unit frames 54a and the length of a short side intersecting the common sides is 2/3 of the length of each of the common sides are arranged without a gap along a side edge of the group of the first unit frames 54a, and rhombic fourth unit frames 54d in which the length of one side is 2/3 of the length of one side of the first unit frame 54a are arranged without a gap along a side edge of the group of the second unit frames 54b and the third unit frames 54c. In this case, a minimum of the area of the unit frame 54 of the groove 53 is 4/9 when a maximum thereof is 1, which is a small change as compared with two examples described later, but this change is preferable in terms of suppressing deformation at an end portion of the absorber 56 on the waist opening WO side due to an elastic member while maintaining the flexibility of the absorber 56.

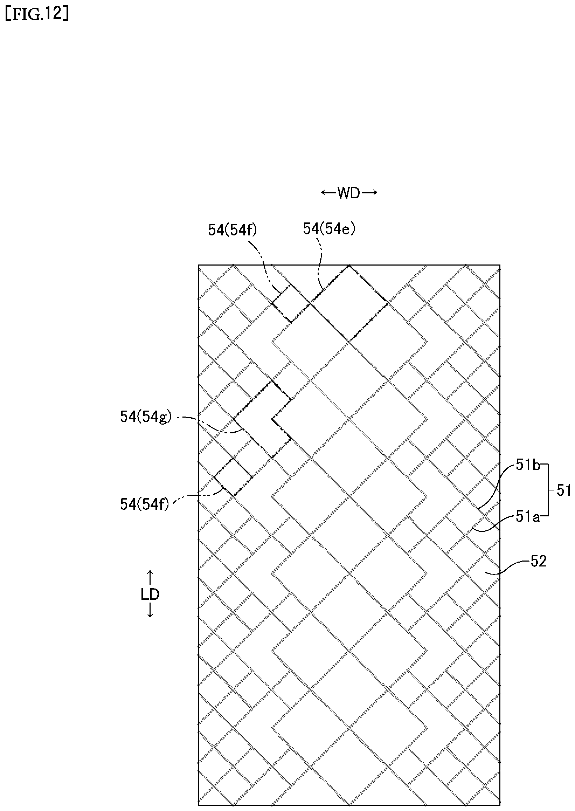

In addition, the pattern of the groove 53 in the edge region 56E of the example illustrated in Fig. 12 is also an oblique lattice shape. In this pattern, rhombic first unit frames 54e having the largest area are arranged without a gap at the center in the width direction WD, rhombic second unit frames 54f in which the length of one side is 1/2 of one side of the first unit frame 54e and L-shaped third unit frames 54g in which the length of each of two long sides is the same as the length of one side of the first unit frame 54e and the length of each of four short sides is 1/2 of the length of one side of the first unit frame 54e are arranged without a gap along a side edge of the group of the first unit frames 54e, and the rhombic second unit frames 54f in which the length of one side is 1/2 of the length of one side of the first unit frame 54e are arranged without a gap along a side edge of the third unit frames 54g. In this case, a minimum of the area of each of the unit frames 54e to 54g is 1/4 when a maximum thereof is 1.

In addition, the pattern of the groove 53 in the edge region 56E of the example illustrated in Fig. 13 is also an oblique lattice shape. In this pattern, rhombic first unit frames 54h having the largest area are arranged without a gap at the center in the width direction WD, rhombic second unit frames 54i in which the length of one side is 1/2 of one side of the first unit frame 54h are arranged without a gap along a side edge of the group of the first unit frames 54h, and rhombic third unit frames 54j in which the length of one side is 1/3 of one side of the first unit frame 54h are arranged without a gap along a side edge of the group of the second unit frames 54i. In this case, a minimum of the area of each of the unit frames 54h to 54j is 1/9 when a maximum thereof is 1.

According to findings of the present inventor, in a case where the interval 56d in the front-back direction LD between an elastic member closest to the edge region 56E of the absorber 56 and the edge region 56E is 15 mm or less (particularly 10 mm or less), an end portion of the absorber 56 on the waist opening WO side is particularly easily deformed. In addition, in this case, when a test piece obtained by cutting a portion of the continuous stretchable band A2 up to 15 mm from the edge region 56E of the absorber 56 to the waist opening WO side from one of the side seals 12A to the other of the side seals 12A is extended to 90% of a maximum width in an unfolded state and then contracted to 70% of the maximum width in the unfolded state, if a contraction force is 0.4 N or more (more preferably 0.5 to 1.5 N), an end portion of the absorber 56 on the waist opening WO side is more easily deformed. Therefore, in these cases, the rigidity of the edge region 56E is preferably increased stepwise or continuously as it goes from the center of the absorber 56 in the width direction WD toward both side edges of the absorber 56 by the above-described pattern change of the groove 53 or the like. Note that, in a case where these conditions are satisfied in one of the front and back bodies, and these conditions are not satisfied in the other body, the rigidity of the edge region 56E can be increased stepwise or continuously as it goes from the center of the absorber 56 in the width direction WD toward both side edges of the absorber 56 by the above-described pattern change of the groove 53 or the like in one body, and the rigidity of the edge region 56E cannot be increased stepwise or continuously as it goes from the center of the absorber 56 in the width direction WD toward both side edges of the absorber 56 by the pattern change of the groove 53 or the like in the other body (that is, the groove 53 is formed in a constant pattern in the width direction WD, or the groove 53 is not formed at all).

### <Experimental Example>

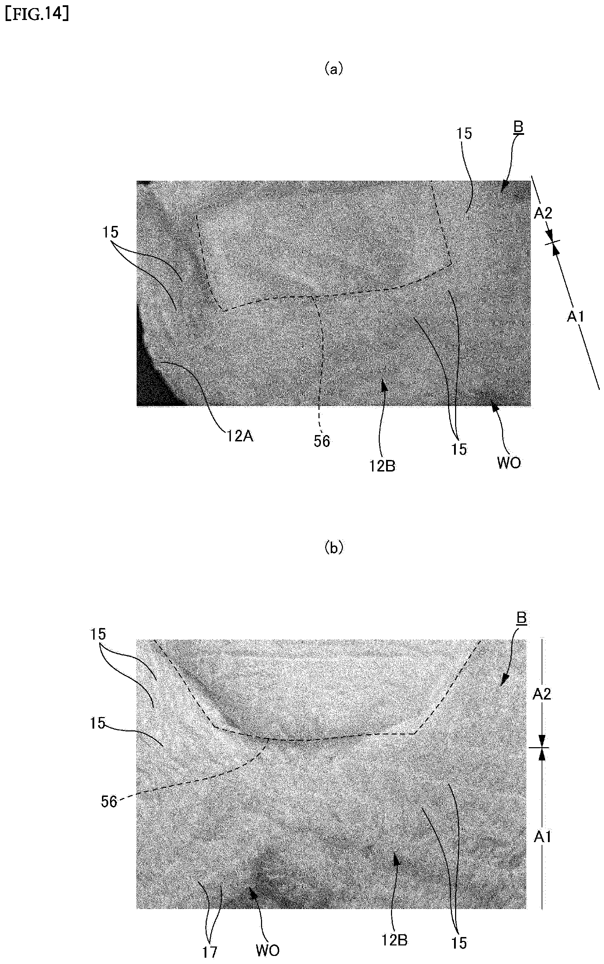

A sample A (pattern illustrated in Figs. 9 to 11) and a sample B (oblique lattice-shaped pattern in which the same unit frame 54 as the maximum unit frame 54a of the sample A is repeated over the entire absorber 56) of the underpants-type disposable diaper illustrated in Figs. 1 to 7, which are common except that the sample A and the sample B had different patterns of the groove 53, were prepared. As a result, in the sample A, deformation at the end portion of the absorber 56 on the waist opening WO side due to the elastic member 15 could be suppressed as illustrated in the natural length state in Fig. 14(a), whereas in the sample B, both corner portions of the absorber 56 on the waist opening WO side selectively contracted in the width direction WD, the intermediate portion in the width direction WD at the end portion of the absorber 56 on the waist opening WO side hardly contracted, and the intermediate portion in the width direction WD at the end portion of the absorber 56 on the waist opening WO side had a shape bulging toward a back surface side (external surface side) as illustrated in the natural length state in Fig. 14(b).

### <Explanation of terms in specification>

The following terms in the specification have the following meanings unless otherwise specified in the specification.
- "Front-back direction" means a direction (longitudinal direction) indicated by a reference character LD in the drawing, "width direction" means a direction (left-right direction) indicated by a reference character WD in the drawing, and the front-back direction and the width direction are orthogonal to each other.
- "Front surface side" means a side closer to a wearer's skin when a diaper is worn. "Back surface side" means a side far from a wearer's skin when a diaper is worn.
- "Front surface" means a surface closer to a wearer's skin when a diaper is worn. "Back surface" means a surface far from a wearer's skin when a diaper is worn.
- "Stretch rate" means a value obtained when a natural length is 100%. For example, a stretch rate of 200% is synonymous with an elongation ratio of 2.
- "Gel strength" is measured as follows. To 49.0 g of artificial urine (mixture of 2% by weight of urea, 0.8% by weight of sodium chloride, 0.03% by weight of calcium chloride dihydrate, 0.08% by weight of magnesium sulfate heptahydrate, and 97.09% by weight of deionized water), 1.0 g of a super absorbent polymer is added, and the resulting mixture is stirred with a stirrer. The gel thus generated is left in a thermohygrostat at 40°C x 60%RH for three hours. Thereafter, the temperature is returned to room temperature, and gel strength is measured with a curdmeter (Curdmeter-MAX ME-500 manufactured by I. Techno Engineering Co., Ltd.).
- "Basis weight" is measured as follows. A sample or a test piece is predried and then left in a test chamber or an apparatus in a standard state (test location is at a temperature of 23 ± 1°C and a relative humidity of 50 ± 2%) so as to have a constant weight. Predrying refers to causing a sample or a test piece to have a constant weight in an environment of a temperature of 100°C. Note that fibers having an official moisture regain of 0.0% do not have to be predried. A sample of 100 mm × 100 mm in size is cut out from a test piece having a constant weight using a template for sampling (100 mm × 100 mm). The weight of the sample is measured. The weight is multiplied by 100 to calculate the weight per square meter to be used as a basis weight.
- "Thickness" of a thick member such as the absorber 56, the absorbent element 50, or the bottom portion 51 of the groove is measured using a thickness meter (Peacock, dial thickness gauge, model H (measurement range: 0 to 10 mm), circular terminal with a measurement area diameter of 10 mm, measurement force: about 1.7 N, pressure: about 21.7 KPa)) manufactured by Ozaki Mfg. Co., Ltd. and making a sample and the thickness meter horizontal.
- "Thickness" of a thin sheet such as a nonwoven fabric is automatically measured under conditions that a load is 0.098 N/cm² and a pressing area is 2 cm² using an automatic thickness meter (KES-G5 handy compression measuring program).
- Water absorption capacity is measured in accordance with JIS K7223-1996 "Test method for water absorption capacity of super absorbent polymer".
- Water absorption speed is "time to end point" when JIS K7224-1996 "Test method for water absorption speed of super absorbent polymer" is performed using 2 g of super absorbent polymer and 50 g of physiological saline.
- "Unfolded state" means a flatly unfolded state without contraction (including any contraction such as contraction by an elastic member) or slack.
- "Maximum elongation" means a maximum value of elongation in a stretchable direction in a stretchable region (that is, elongation at an elastic limit, which is equal to elongation in an unfolded state), and is represented by a percentage when a natural length is 100%.
- The size of each portion means a size not in a natural length state but in an unfolded state unless otherwise specified.
- When environmental conditions in a test and a measurement are not described, the test and the measurement are performed in a test room or an apparatus in a standard state (test location is at a temperature of 23 ± 1°C and a relative humidity of 50 ± 2%).

### Industrial Applicability

The present invention can be used for an underpants-type disposable wearable article such as the underpants-type disposable diaper of the above example.

### Reference Signs List

- 11: Liquid impervious sheet
- 12A: Side seal
- 12B: Back outer member
- 12F: Front outer member
- 12H: Inner sheet layer
- 12S: Outer sheet layer
- 12X: Non-stretchable region
- 13: Cover nonwoven fabric layer
- 14: Gluteal cover portion
- 15: Under-waist elastic member
- 16: Gluteal cover elastic member
- 17: Waist elastic member
- 30: Top sheet
- 50: Absorbent element
- 51: Bottom portion
- 52: Non-compressed portion
- 53: Groove
- 54: Unit frame
- 56: Absorber
- 56E: Edge region
- 56M: Portion having a minimum width
- 56N: Portion having a minimum width
- 56V: Widened portion
- 58: Wrapping sheet
- 60: Side flap
- 61: Front surface portion
- 62: Back surface portion
- 64: Liquid impervious sheet
- 65: Nonwoven fabric absent portion
- 66: Side nonwoven fabric
- 66r: Folded-back portion
- 67: Fixed portion
- 70: Side non-stretchable region
- 80: Planar gather region
- 200: Inner member
- 201: Fixing region
- 631 to 633: Gather elastic member
- 631: First gather elastic member
- 632: Second gather elastic member
- 633: Third gather elastic member
- A1: Intermittent stretchable band
- A2: Continuous stretchable band
- B: Back body
- BT: Back lower torso portion
- F: Front body
- FT: Front lower torso portion
- HM1: First hot melt adhesive
- HM2: Second hot melt adhesive
- HM3: Third hot melt adhesive
- L: Intermediate region
- LD: Front-back direction
- M: Crotch portion
- T: Lower torso region
- U: Under-waist portion
- W: Waist portion
- WD: Width direction
- WO: Waist opening
- d1: First interval
- d2: Second interval
- d3: Third interval
- w1: First distance
- w2: Second distance
- w3: Length of fixed portion in width direction
- w4: Third distance
- w5: Fourth distance.

## Claims

1. An underpants-type disposable wearable article comprising:
an outer member having a front lower torso portion, a back lower torso portion, and side seals formed by bonding both side portions of the front lower torso portion and both side portions of the back lower torso portion to each other;
an inner member extending from the front lower torso portion to the back lower torso portion through a crotch portion;
a waist opening formed by a front edge of the front lower torso portion and a back edge of the back lower torso portion; and
leg openings formed on both sides of the inner member, wherein
the outer member has a fixing region where the inner member is fixed to at least the front lower torso portion and the back lower torso portion,
the inner member includes an absorber that continues from a position overlapping the front lower torso portion to a position overlapping the back lower torso portion,
the absorber has an edge region including the whole of an edge on a waist opening side and a portion adjacent thereto on a crotch portion side in a width direction, and the entire edge region is located in the fixing region of the inner member and fixed to the outer member,
in the front lower torso portion and the back lower torso portion, a first region including a portion overlapping the absorber and a portion between the overlapped portion and the side seals is an intermittent stretchable band having a non-stretchable region provided at least at an intermediate portion in the width direction in the portion overlapping the absorber and a stretchable region provided between the non-stretchable region and each of the side seals, and a second region located between the intermittent stretchable band and the waist opening is a continuous stretchable band in which a stretchable region is continuous in the width direction from one of the side seals to the other of the side seals,
the stretchable region in the intermittent stretchable band and the continuous stretchable band incorporates an elastic member and is stretchable between a natural length state in which the stretchable region contracts in the width direction together with the elastic member and a stretched state in which the stretchable region extends in the width direction together with the elastic member,
a groove recessed from a surface of the absorber into the absorber and having a bottom portion compressed in a thickness direction is formed in a pattern continuous in the width direction over the entire edge region in the width direction, and
in the edge region, a thickness of the bottom portion of the groove is constant and an area ratio of the bottom portion of the groove increases, the area ratio of the bottom portion of the groove is constant and the thickness of the bottom portion of the groove decreases, or the area ratio of the bottom portion of the groove increases and the thickness of the bottom portion of the groove decreases as it goes from a center of the absorber in the width direction toward both side edges of the absorber.

2. The underpants-type disposable wearable article according to claim 1, wherein
the edge region is a region where the groove is continuous in a lattice shape, and
each of a thickness of the bottom portion of the groove and a width of the bottom portion of the groove in the edge region is constant, and an area of a unit frame including the groove in the edge region gradually decreases as it goes from a center in the width direction toward both side edges.

3. The underpants-type disposable wearable article according to claim 1 or 2, wherein
a plurality of the elastic members in the continuous stretchable band are attached at intervals in a front-back direction and are elongated elastic members in the width direction, and
an interval between the elongated elastic member closest to the edge region of the absorber and the edge region in a front-back direction is 15 mm or less.

4. The underpants-type disposable wearable article according to claim 3, wherein
when a test piece obtained by cutting a portion of the continuous stretchable band up to 15 mm from the edge region of the absorber to the waist opening side from one of the side seals to the other of the side seals is extended to 90% of a maximum width in an unfolded state and then contracted to 70% of the maximum width in the unfolded state, a contraction force is 0.4 N or more.

5. The underpants-type disposable wearable article according to claim 1 or 2, wherein
the absorber includes one or more layers formed by mixing and accumulating pulp fibers and super absorbent polymer particles,
a basis weight of the pulp fibers in the absorber is 80 to 450 g/m²,
a weight ratio of pulp fibers : super absorbent polymer particles in the absorber is 20 : 80 to 80 : 20,
a maximum value of a thickness of the absorber is 1 to 20 mm,
a thickness of the bottom portion is 5 to 40% of a maximum value of the thickness of the absorber, and
a width of the bottom portion is 1 to 3 mm.

6. The underpants-type disposable wearable article according to claim 1 or 2, wherein
the groove is formed in an oblique lattice shape including a first portion inclined clockwise by 40 to 60° in plan view with respect to a front-back direction and a second portion inclined counterclockwise by 40 to 60° in plan view with respect to the front-back direction.
